(19) Europäisches Patentamt
European Patent Office
Office européen des brevets

(11) Veröffentlichungsnummer: **0 117 436**
**B1**

(12) # EUROPÄISCHE PATENTSCHRIFT

(45) Veröffentlichungstag der Patentschrift: **20.05.87**

(51) Int. Cl.⁴: **G 01 N 33/76**, G 01 N 33/546

(21) Anmeldenummer: **84100793.3**

(22) Anmeldetag: **26.01.84**

(54) **Immunologisches Latex-Agglutinationsverfahren.**

(30) Priorität: **31.01.83 DE 3303083**

(43) Veröffentlichungstag der Anmeldung: **05.09.84 Patentblatt 84/36**

(45) Bekanntmachung des Hinweises auf die Patenterteilung: **20.05.87 Patentblatt 87/21**

(84) Benannte Vertragsstaaten: **AT BE CH DE FR GB IT LI LU NL SE**

(56) Entgegenhaltungen:
**EP-A-0 013 032**
**EP-A-0 038 181**
**DE-A-2 529 937**
**GB-A-2 030 293**
**GB-A-2 042 170**

**Die Akte enthält technische Angaben, die nach dem Eingang der Anmeldung eingereicht wurden und die nicht in dieser Patentschrift enthalten sind.**

(73) Patentinhaber: **BEHRINGWERKE Aktiengesellschaft, Postfach 1140, D-3550 Marburg 1 (DE)**

(72) Erfinder: **Toth, Tibor, Dr., Gründeberg 1, D-3550 Marburg (DE)**

(74) Vertreter: **Meyer- Dulheuer, Karl- Hermann, Dr., HOECHST Aktiengesellschaft Zentrale Patentabteilung Postfach 80 03 20, D-6230 Frankfurt/Main 80 (DE)**

**EP 0 117 436 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein Latex-Agglutinationsverfahren zum Nachweis oder zur Bestimmung eines der Partner einer Antigen-Antikörper-Reaktion in Gegenwart eines Zusatzes, der Störungen durch unspezifische Reaktionen verhindert sowie die Herstellung einer dafür geeigneten Mittels.

In jüngerer Zeit werden in zunehmendem Maße immunologische Diagnose-Methoden wegen ihrer hohen Spezifität und Empfindlichkeit angewendet. Von diesen Methoden hat die Latex-Agglutionationsreaktion, bei der die Latex-Teilchen entweder mit einem Antigen oder einem Antikörper sensibilisiert sind, den Vorteil, daß die Handhabung sehr einfach ist und die Ergebnisse in kurzer Zeit erhälten werden können.

Diese Methoden können zu Fehldiagnosen führen, da reaktionsfremde Substanzen, die im Urin oder in Körperflüssigkeiten wie Seren, Plasma oder Synovia enthalten sind, unspezifiche Agglutinationsreaktionen verursachen können. Um derartige Störfaktoren zu entfernen, werden Urine zum Beispiei bei Schwangerschaftsdiagnosen über Zellstoff-Filterpapiere, Watte, Celluloseacetat, Polyacrylnitril, Carboxymethylcellulose oder andere Faserstoffe filtriert. Jedoch ist keines dieser Materialien völlig befriedigend. Beispielsweise vermögen Filterpapiere und Watte zwar Trübstoffe zu entfernen, haben jedoch den Nachteil, daß auch das nachzuweisende HCG absorbiert wird.

Aus DE-A- 29 51 672, veröffentlicht am 3. Juli 1980, ist bekannt, die zu untersuchenden Flüssigkeiten mit Fasern aus einem Kationenaustauscherharz vom Carbonsäure-TYP vorzubehandeln. Die Herstellung geeigneter Fasern ist jedoch schwierig.

In DE-A 30 02 973 ist beschrieben, eine Latex-Agglutinationsreaktion in Gegenwart eines Harnstoff- oder Formamidderivats durchzuführen. Diese können die oben geschilderten Schwierigkeiten zwar verringern, jedoch nicht beheben.

Es wurde nun überraschenderweise geunden, daß die vorstehend genannten technischen Schwierigkeiten verhindert werden können, indem die Latex-Agglutinationsreaktion in Gegenwart einer Verbindung der allgemeinen Formel I

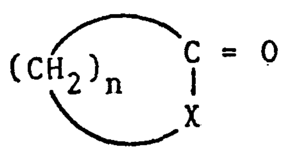

(I)

worin X = O oder NH
und n = 2 - 9 ist,
ausgeführt wird.

Gegenstand der Erfindung ist deshalb ein Latex-Agglutinationsverfahren zum Nachweis oder zur Bestimmung eines Partners in einer Antigen-Antikörper-Reaktion mittels des entsprechenden partners, dadurch gekennzeichnet, daß man die Partner in Gegenwart einer Verbindung der Formel I und den angegebenen Definitionen miteinander reagieren läßt.

Beispiele geeigneter Verbindungen sind: Butyrolacton, Valerolacton, Caprolacton, Pyrrolidon, Valerolactam, Caprolactam. Ein hohes Dipolmoment und gute Lösungseigenschauten, wie sie das Butyrolactam (Pyrrolidon) besitzt, sind von Vorteil. Die Verbindungen können allein oder in Kombinationen verwendet werden. Besonders vorteilhaft ist die Kombination Butyrolacton/Pyrrolidon und Pyrrolidon/Caprolactam.

Die Erfindung ist auf alle Reaktionen zum Nachweis von immunologisch aktiven Substanzen, die in Körperflüssigkeiten wie beispielsweise Serum, Plasma oder Synovia und insbesondere im Urin vorhanden sein können, auf der Grundlage einer Latex-Agglutination anwendbar. Als Beispiele für die zu bestimmenden immunologisch aktiven Substanzen seien Serumproteine wie Myoglobin, Hämoglobin, $ß_2$-Mikroglobulin und $\alpha_1$-Mikroglobulin, Hormone wie HCG (Human Chorion Gonadotropin) oder Amylase genannt. Die Verbindungen gemäß Formel I können in das Latex-Agglutinationsreaktions-System eingearbeitet werden, indem man sie dem diagnostischen Mittel, das die mit einem der Partner einer immunologischen Reaktion beladenen (sensibilisierten) Latexteilchen enthält, zusetzt, oder sie können, gegebenenfalls in einem Lösungsmittel, der Testprobe zugemischt werden.

Als Latexteilchen, die mit einer immunologisch aktiven Substanz sensibilisiert werden, kommen alle Latices in Frage, die für den Latex-Agglutinationstest geeignet sind. Als Beispiele seien genannt: Latices aus Homopolymeren und Copolymeren des Styrols oder seiner Derivate wie Methylstyrol, Ethylstylol oder Chlorstyrol, der Acrylsäure oder ihrer Ester wie Methylacrylat oder Ethylacrylat, der Methacrylsäure oder ihrer Derivate wie Ethylmethacrylat, Acrylnitril oder Acrylamid, von Dienen wie Butadien, Chloropren oder Isopren, des Vinylchlorids, Vinylidenchlorids oder Vinylacetats. Von diesen werden die Latices der Homopolymeren oder Copolymeren von Styrol, Acrylsäure oder Methylmethacrylat vorteilhaft verwendet. Bevorzugt werden Latices mit einer Teilchengröße von etwa 0,1 bis etwa 1 µm, insbesondere Teilchen einer Größe von etwa 0,1 bis etwa 0,6 µm.

Die Sensibilisierung der Latexteilchen mit einer immunologisch aktiven Substanz, also einem Antigen oder Antikörper, kann nach einer bekannten Methode durchgeführt werden. Die Bedingungen variieren in einem gewissen Maß in Abhängigkeit von den physikalisch-chemischen Eigenschaften der sensibilisierenden Substanz und der Latexteilchen. Wenn die Latexteilchen beispielsweise mit einem Antikörper sensibilisiert werden sollen, wird zweckmäßig wie folgt gearbeitet: Aus einem diesen Antikörper enthaltenden Antiserum

wird in üblicher Weise eine Gammaglobulinfraktion ausgefällt, die in einer Konzentration von 0,01 bis 4 g/100 ml in einem 0,005 bis 0,2 molaren Puffer mit einem pH-Wert von 7 bis 9 gelöst wird. Als Puffer wird zweckmäßig ein Glycin-NaCl-, Phosphat-, Imidazol- oder Borat-Puffer verwendet. Bevorzugt wird eine 0,15 mol/l Glycin-NaCl-Pufferlösung von pH 8,2. Eine Suspension von Latexteilchen mit einer Konzentration von 0,1 bis 10 g/100 ml wird der Gammaglobulin-Lösung zugesetzt und die Mischung 1 bis 6 Stunden bei Raumtemperatur oder 0,5 bis 3 Stunden bei 35 bis 56° C stehengelassen oder gerührt. Die Suspension wird danach zentrifugiert.

Die mit Antikörpern oder Antigen beladenen Latexpartikel werden in einer Pufferlösung, bevorzugt einer Glycin-Natriumchlorid- oder Imidazol-Pufferlösung, insbesondere einer 0,1 bis 0,3 molaren Imidazol-Pufferlösung, die bis 30 g/100 ml, vorzugsweise 10 bis 15 g/100 ml, einer Verbindung der Formel I enthalten kann, suspendiert, so daß bevorzugt eine Konzentration von 0,6 bis 1,2 g Latex in 100 ml Suspension erhalten wird.

Eine Verbindung der Formel I kann auch der zu testenden Flüssigkeit zugesetzt werden. Gegebenenfalls kann zusätzlich ein Lösungsmittel für die Verbindung der Formel I verwendet werden.

Die folgenden beispiele erläutern die Erfindung.

**Beispiel 1**

a) Nach dem Stand der Technik hergestelltes Latex-Reagenz, welches als spezifische Antikörper immunadsorptiv gewonnene Antikörper gegen Human-HCG vom Kaninchen (Latex-HCG-Reagenz) enthielt, wurde in folgendem Test eingesetzt (Ic). Die Empfindlichkeit des Reagenzes wurde an einem Standard auf 0,5 bis 1 IU/ml eingestellt.

b) 8 g Pyrrolidon und 7 g Butyrolacton wurden in 100 ml 0,2 molarer Imidazol-Pufferlösung von pH 8,2 gelöst.

c) 1 Tropfen unverdünnter zu prüfender Urin (50 µl) wurde auf ein Feld einer Testplatte gegeben. 25 µl Lösung nach 1b) und 25 µl Latex-HCG-Reagenz wurden zugesetzt. Nach Durchmischen mittels eines Rührstäbchens wurde die Testplatte rotierend bewegt und nach 3 Minuten auf Agglutination geprüft.

**Beispiel 2**

a) Das Latex-Reagenz wurde wie bei Ia) hergestellt.

b) 5 g Caprolactam und 12 g Pyrrolidon wurden in 100 0,15 molarer Glycin-NaCl-Pufferlösung gelöst.

c) 1 Tropfen unverdünnter zu prüfender Urin (50 µl) wurde auf ein Feld einer Testplatte gegeben. 25 µl Lösung nach 2b) und 25 u1 Latex-HCG-Reagenz wirden zugesetzt. Nach Durchmischen mittels eines Rührstäbchens wurde die Testplatte rotierend bewegt und nach 3 Minuten auf Agglutination geprüft.

| Probenzahl | HCG-Gehalt | Herkunft | Zahl der pos. Stand der Technik* | Proben erfindungs-gemäß |
|---|---|---|---|---|
| 3 | 0 | Männer | 1 | 0 |
| 2 | 0 | Nichtschwagnere | 1 | 0 |
| 5 | + | Schwangere ** | 5 | 5 |

*)Suspension sensibilisierter Latexpartikel in Glycin-NaCl-Puffer ohne Verbindung der Formel I
**) gesicherte Schwangerschaften

Die Tabelle zeigt, daß das Reagenz nach dem Stand der Technik in 5 Proben (3 von Männern, 2 von Nichtschwangeren) 2 falsch positive Ergebnisse lieferte, während nach dem erfindungsgemäßen Verfahren keine falsch positiven Resultate gefunden wurden.

**Patentansprüche**

1. Latex-Agglutinationsverfahren zum Nachweis oder zur Bestimmung eines Partners in einer Antigen-Antikörper-Reaktion mittels des entsprechenden Partners, dadurch gekennzeichnet, daß man die Partner in Gegenwart einer Verbindung der Formel I

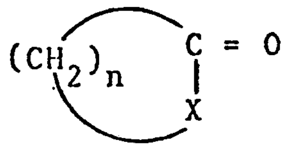

(I)

worin X = 0 oder NH
und n = 2 - 9 ist,
miteinander reagieren läßt.

2. Verfahren nach Anspruch 1, dadurch gekennzeichnet, daß die Verbindung der Formel 1 Butyrolacton, Valerolacton, Caprolacton, Pyrrolidon, Valerolactam und/oder Caprolactam ist.

3. Verfahren nach Anspruch 1, dadurch gekennzeichnet, daß als Verbindung der Formel I ein Gemisch von Butyrolacton und Pyrrolidon oder Pyrrolidon und Caprolactam verwendet wird.

4. Verwendung einer Verbindung der Formel I in Anspruch 1 zur Herstellung eines Mittel zum Nachweis von Human Chorion Gonadotropin mittels Latex-Agglutinationsrreaktion.

**Claims**

1. A latex agglutination procedure for detecting or determining a participant in an antigen-antibody reaction using the corresponding participant, which procedure comprises allowing the participants to react together in the presence of a compound of the formula I

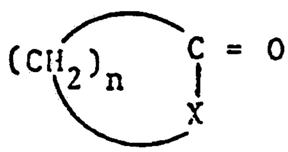

(I)

in which X is O or NH, and
n is 2 - 9.

2. The procedure as claimed in claim 1, wherein the compound of the formula I is butyrolactone, valerolactone, caprolactone, pyrrolidone, valerolactam and/or caprolactam.

3. The procedure as claimed in claim 1, wherein a mixture of butyrolactone and pyrrolidone or of pyrrolidone and caprolactam is used as the compound of the formula I

4. The use of a compound of the formula I in claim 1 in a process for manufacturing an agent for detectinug human chorionic gonadotropin by means of a latex agglutination reaction.

**Revendications**

1. Procédé d'agglutination de latex pour la mise en évidence ou le dosage d'un partenaire dans une réaction antigène-anticorps au moyen du Partenaire correspondant, caractérisé en ce qu'on fait réagir le partenaire en présence d'un composé répondant à la formule I

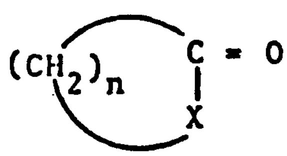

(I)

dans laquelle:
K = O ou NH, et
n = 2 - 9.

2. Procédé suivant la revendication 1, caractérisé en ce que le composé répondant à la formule I est la butyrolactone, la valérolactone, la caprolactone, la pyrrolidone, le valérolactame et/ou le caprolactame.

3. Procédé suivant la revendication 1, caractérisé en ce qu'on utilise comme composé répondant à la formule 1 un mélange de butyrolactone et de pyrrolidone ou de pyrrolidone et de caprolactame.

4. Utilisation d'un composé de formule I défini à la revendication 1 pour la préparation d'un agent pour la détection de la gonadotropine chorionique humaine au moyen d'une réaction d'agglutination de latex.